# EUROPEAN PATENT SPECIFICATION

(11) **EP 0 586 929 B1**
(45) Date of publication and mention of the grant of the patent: **07.09.1994**
(21) Application number: 93113101.5
(22) Date of filing: 16.08.1993
(51) Int. Cl.: A61K 7/06

(54) **Hair treatment composition and process**
Haarbehandlungsmittel und Verfahren
Composition pour le traitement des cheveux et procédé

(30) Priority: 17.08.1992 DE 4227203
(43) Date of publication of application: 16.03.1994
(73) Proprietor: Kao Corporation, Chuo-Ku Tokyo 103 (JP)
(72) Inventor: Jendryssek-Pfaff, Madeleine, D-97708 Aschach (DE); Onitsuka, Satoshi, D-64295 Darmstadt (DE)
(74) Representative: Patentanwälte Zellentin & Partner

(56) References cited:
- EP-A- 0 027 730
- DE-A- 2 317 140
- US-A- 3 723 324
- PATENT ABSTRACTS OF JAPAN vol. 6, no. 186 (C-126)(1064) 22 September 1982
- PATENT ABSTRACTS OF JAPAN vol. 11, no. 206 (C-433)(2653) 3 July 1987

## Description

This invention comprises a composition and a process for the treatment of hair providing an improved conditioning effect compared with conventional compositions.

Hair conditioning compositions have been known since a long time and are still very popular among users, particularly female consumers. They are usually applied after hair cleansing, dyeing or waving processes. Above all it is their task to restore the hair structure, improve lustre and touch thereof, ease combability of wet and dry hair, and provide a lasting hair style.

The number of active substances suggested for this purpose is immense; reference is, e.g., made to the monography of K.Schrader, "Grundlagen und Rezepturen der Kosmetika", 2nd Edition (1989, Hüthig Buchverlag, Heidelberg), pp. 722 to 781.

Known hair conditioning compositions still require improvement, in spite of the multitude of known and suggested formulations, especially with regard to their ability to penetrate into the hair.

It has now been found that an excellent conditioning effect can be achieved if the hair is treated with a composition being obtained by admixture of two components kept separate until application, whereby one composition (A) is essentially anhydrous and contains a physiologically compatible salt that develops heat upon admixture with water, and at least one thickening agent, and a second composition (B) contains at least one polyalcohol which is liquid at 25°C and selected from the group of polyethyleneglycol, polypropyleneglycol, glycerol and (or) diglycerol, whereby these compositions are, under addition of water, mixed while heat is generated, and the warm gel thus obtained is applied onto human hair and rinsed after processing.

DE-A 23 17 140 discloses a cosmetic two-component mixture of two separately packed components, the first one comprising a customary aqueous cosmetic composition, and the second one comprising a salt or a salt-containing mixture giving a temperature increase upon admixture with the first component by exothermic hydratation.

According to the description, the salt component may also contain solid components which are not suitable for temperature increase, e.g. finely divided silica, for improvement of flowability.

The use of polyalcohols being liquid at 25°C which is essential for the present invention is not mentioned, because the problem to be solved there is different from that of the invention.

However, comparative tests have shown that, without the presence of polyalcohols, even with high salt concentration, a sufficient heat preservation on the hair cannot be effected. Just the opposite:
The optimal temperature is already reached after short time; afterwards, a fast temperature decrease occurs.

The abstract of JP-A 57 099 514 describes cosmetics containing an inorganic, heat-generating material in contact with water.

These products differ from the present invention insofar as they do not contain polyalcohols being liquid at 25°C in a composition being separated until use from a water-free composition containing a metal salt and a thickening agent.

As shown by comparative testing, the pulverulent combination of metal salt and thickening agent is necessary to get a sufficient viscosity increase compared to compositions where the thickening agent is only present in the liquid phase.

Without sticking to a certain theory, it is believed that the heat treatment essentially increases the penetration of the active ingredients into the hair, which may either the salts developing beat when mixed with water, especially if these salts are bivalent metal chlorides according to a preferred embodiment of the invention, such as calcium chloride, magnesium chloride or zinc chloride, acting as conditioning substances themselves, or the additionally used hair conditioning substances known per se.

As indicated above, the physiologically compatible salt which develops heat when mixed with water is preferably a bivalent metal chloride.

Such bivalent metal chlorides are in particular calcium chloride, magnesium chloride and (or) zinc chloride which also have an additional hair conditioning effect.

Other suitable salts are aluminum chloride, sodium sulfate, sodium carbonate, or beryllium chloride.

The quantity of the metal chloride required to develop heat depends on the desired temperature of the ready-to-use composition, which is about 40 to 60°C, preferably about 50°C.

The same applies for the thickening agent present in the anhydrous composition in admixture with the heat developing salt, whereby generally the proportion of salt : thickening agent should be about 1 : 1 to 50 : 1, particularly about 2 to 5 : 1.

Suitable thickening agents in particular are various cellulose derivatives such as methyl cellulose, hydroxyethyl cellulose, hydroxypropyl cellulose, natural gums such as xanthan gum, guar gum or pectin; but also inorganic thickening agents, e.g. aluminium silicate and magnesium aluminium silicates such as montmorillonite or silica may be used.

The essential component of the composition (B) which is kept separate from the composition (A) containing the anhydrous salt, is a polyalcohol being in liquid state at 25°C, selected from the group of polyethyleneglycol, polypropyleneglycol, glycerol and (or) diglycerol.

The preferred proportion of the heat developing salt when mixed with water in relation to polyalcohol in the ready-to-use mixture is between about 1 : 1 and 1 : 8, especially 1 : 2 and 1 : 6.

A polyethyleneglycol with a molecular weight between about 200 and 600, especially about 400 and about 600, is used as preferred polyalcohol.

The polyalcohol in the ready-to-use composition has a dual function: Firstly it serves as a carrier, and secondly as a thermal reservoir to maintain the heat developed by the mixture of the metal salt with water within the final viscous product for a reasonable time.

This function is best fulfilled by polyethyleneglycol, particularly with a molecular weight between 200 and 600, since it generates additional heat; other suitable polyalcohols such as polypropyleneglycol and glycerol or diglycerol which are preferably used in admixture with polyethyleneglycol do not develop heat in combination with water, but are also suitable to act as a thermal reservoir.

Higher oligoglycerols such as tri-, tetra- and pentaglycerols may be used in admixture with the polyalcohols mentioned above, provided the mixture is liquid at a temperature of 25°C.

The compositions according to the invention may be applied onto the hair and have there a conditioning effect as already described, in particular when containing bivalent metal chlorides such as calcium chloride, magnesium chloride and (or) zinc chloride (wherefrom calcium chloride is preferred).

It is however expedient and advantageous to use additional hair conditioning substances known per se.

In particular the known long-chain quaternary ammonium compounds or cationic polymers are mentioned as such.

Further film-forming ampholytic, nonionic and (or) anionic polymers may be used for this purpose, too, moreover oils, fats, volatile and non-volatile silicones, humectants, amino acids, panthenol, phytantriol, naphthaline sulfonic acid, etc.

Apart from active agents, auxiliaries may also be used, e.g., anionic, nonionic or amphoteric surfactants, preservatives, etc.

The relevant performance of these compounds and their concentrations of use are known in the art, see, e.g., K.Schrader, l.c., for that reason a specific enumeration thereof is not necessary.

The admixture of these hair conditioning compounds may take place either to composition (A) or to composition (B); if they are present in liquid form or in solution or dispersion, composition (B) will be the more suitable part for admixture.

The composition according to the invention may be applied as follows:
A receptacle, e.g. a vial, comprising the polyalcoholic composition (B), preferably in combination with hair conditioning substances and optionally with surfactants (about 5 to 20g), is filled up with water (about 30 ml), and the solution thus obtained is added to the anhydrous composition (A) comprising an anhydrous salt which generates heat in combination with water, and thickening agents (about 5g in a proportion of about 9 : 1), and mixed. A temperature from about 50 to 55°C is generated under gel formation. This composition is applied onto wet hair and allowed to act for about 10 minutes, whereby the temperature slowly decreases to about 40°C. Thereafter the treated hair is rinsed.

The sequence of admixture of the compositions (A) and (B) may also be reversed.

The composition (B) comprising the polyalcohol may contain additionally a water-free alcohol, such as 1,3- or 1,4-butanediol, benzyl alcohol, or benzyloxyethanol, 1-methoxypropanol, or dipropyleneglycol monomethylether to improve penetration.

The proportion of penetration promoter may be between 0 and 25% by weight of the composition (B).

A general formula of the compositions according to the invention is defined about as follows:

| | |
|---|---|
| Calcium chloride, magnesium chloride and (or) zinc chloride | 1 - 70, preferably 1 - 25, most preferred 3 - 15% by wt.; |
| Polyethyleneglycol (MG 200 - 600) | 1 - 60, preferably 1 - 40, most preferred 5 - 30% by wt.; |
| Thickening agent | 0.5 - 15, preferably 1 - 10, most preferred 1.5 - 8% by wt; |
| Water | @ 100% by wt. |

These percentages refer to the ready-to-use mixture.

The metal salt proportions obviously depend on the fact whether or not they are free from crystal water which are preferred, or crystal water containing salts. In the first case the levels are lower, in the second case higher.

The following examples further illustrate the invention:

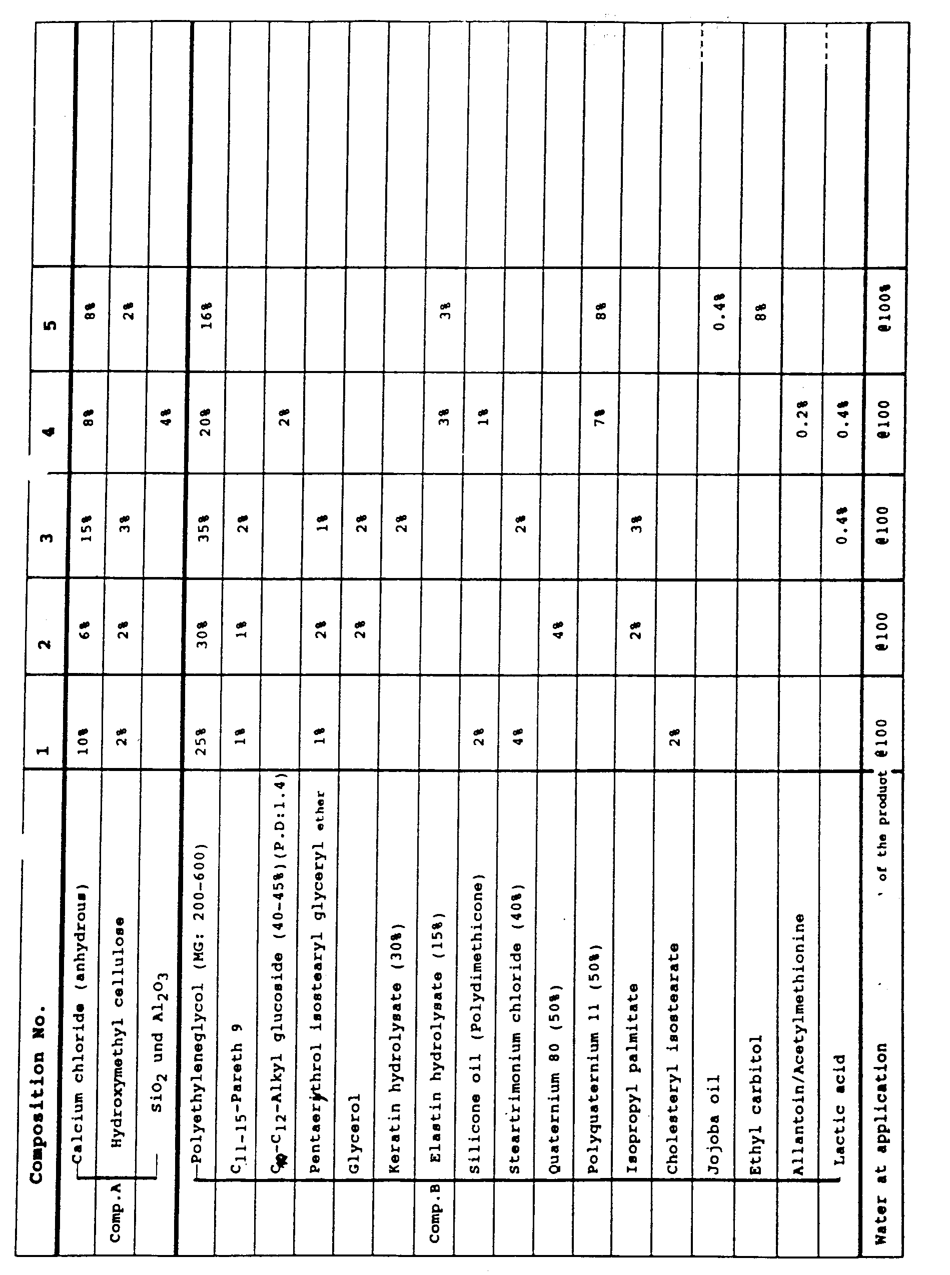

The hair treated with these compositions presented volume, firm hold but relaxed touch and natural gloss.

## Claims

1. Composition for the treatment of human hair, comprising at least two compositions kept separate until application which lead to an increased viscosity upon admixture in the presence of water while developing heat, wherein one composition (A) contains an essentially anhydrous mixture of a physiologically compatible salt developing heat when mixed with water, and at least one thickening agent, and a further composition (B) kept separate until application contains at least one polyalcohol being liquid at 25°C selected from the group polyethyleneglycol, polypropyleneglycol, glycerol and (or) diglycerol.

2. Composition according to claim 1, characterized in that it contains a physiologically compatible bivalent metal chloride which develops heat when mixed with water.

3. Composition according to claim 2, characterized in that it contains calcium chloride, magnesium chloride and (or) zinc chloride as a bivalent metal chloride.

4. Composition according to one of the preceding claims, characterized in that composition (B) contains a polyethyleneglycol having a molecular weight between 200 and 600.

5. Composition according to claim 4, characterized in that composition (B) contains a polyethyleneglycol having a molecular weight from 400 to 600.

6. Composition according to one of the preceding claims, characterized in that it contains as thickening agent a cellulose derivative and (or) a natural gum derivative.

7. Composition according to one of claims 1 to 5, characterized in that it contains as thickening agent silica and (or) a aluminium silicate.

8. Composition according to claim 6, characterized in that it contains as thickening agent xanthan gum and (or) guar gum.

9. Composition according to one of the preceding claims, characterized in that in the final mixture to be applied onto the hair the proportion of physiologically compatible salt developing heat when mixed with water to the liquid polyalcohol is between 1 : 1 and 1 : 8.

10. Composition according to claim 9, characterized in that the weight proportion of the salt developing heat when mixed with water to the liquid polyalcohol is between 1 : 2 to 1 : 6.

11. Composition according to one of the preceding claims, characterized in that it contains additionally at least one hair care agent.

12. Composition according to claim 11, characterized in that it contains a film-forming agent.

13. Composition according to claim 11, characterized in that it contains a hair conditioning substance.

14. Composition according to claim 11, characterized in that it contains natural and synthetic fats and oils.

15. Composition according to one of the preceding claims, characterized in that the polyalcohol containing composition (B) comprises a penetration promoter.

16. Composition according to claim 15, characterized in that it contains 1,3-butanediol, 1,4-butanediol, benzyl alcohol and (or) benzyloxyethanol as penetration promoter.

17. Process for the treatment or hair, characterized in that an essentially anhydrous composition (A) containing a physiologically compatible salt which develops heat upon admixture with water and at least one thickening agent is mixed with water and with a composition (B) containing at least one polyalcohol which is liquid at 25°C and selected from the group polyethyleneglycol, polypropyleneglycol and di-, tri- or polyglycerol according to claims 1 to 16, generating heat under viscosity increase, whereafter the gel developed thereby is applied onto human hair and rinsed after processing.

## Patentansprüche

1. Mittel zur Haarbehandlung, bestehend aus bis zur Anwendung voneinander getrennt gehaltenen Zusammensetzungen, die beim Vermischen in Gegenwart von Wasser unter Wärmeentwicklung eine Viskositätserhöhung ergeben, wobei mindestens eine Zusammensetzung (A) ein im wesentlichen wasserfreies Gemisch aus einem physiologisch verträglichen, beim Vermischen mit Wasser Wärme entwickelnden Salz und mindestens einem Verdickungsmittel enthält, und mindestens eine weitere, davon bis zur Anwendung getrennt gehaltene Zusammensetzung (B) mindestens einen bei 25 °C flüssigen Polyalkohol aus der Gruppe Polyethylenglycol, Polypropylenglycol, Glycerin und/oder Diglycerin enthält.

2. Mittel nach Anspruch 1, dadurch gekennzeichnet, daß es als physiologisch verträgliches, beim Vermischen mit Wasser Wärme entwickelndes Metallsalz ein zweiwertiges Metallchlorid enthält.

3. Mittel nach Anspruch 2, dadurch gekennzeichnet, daß es als zweiwertiges Metallchlorid Calciumchlorid, Magnesiumchlorid und/oder Zinkchlorid enthält.

4. Mittel nach einem der vorhergehenden Ansprüche, dadurch gekennzeichnet, daß die Zusammensetzung (B) ein Polyethylenglycol mit einem Molgewicht zwischen 200 und 600 enthält.

5. Mittel nach Anspruch 4, dadurch gekennzeichnet, daß die Zusammensetzung (B) ein Polyethylenglycol mit einem Molekulargewicht von 400 bis 600 enthält.

6. Mittel nach einem der vorhergehenden Ansprüche, dadurch gekennzeichnet, daß es als Verdickungsmittel ein Cellulosederivat und/oder ein natürliches Gummiderivat enthält.

7. Mittel nach einem der Ansprüche 1 bis 4, dadurch gekennzeichnet, daß es als Verdickungsmittel eine Kieselsäure und/oder ein Aluminiumsilikat enthält.

8. Mittel nach Anspruch 5, dadurch gekennzeichnet, daß es als Verdickungsmittel Xanthan Gum und/oder Guar Gum enthält.

9. Mittel nach einem der vorhergehenden Ansprüche, dadurch gekennzeichnet, daß in der gebrauchsfertig auf das Haar aufzubringenden Mischung das Verhältnis von physiologisch verträglichem, beim Zusammenbringen mit Wasser Wärme entwikkelndem Salz zu dem flüssigen Polyalkohol zwischen 1 : 1 und 1 : 8 liegt.

10. Mittel nach Anspruch 9, dadurch gekennzeichnet, daß das Gewichtsverhältnis der Zusammensetzung (B) beim Zusammenbringen mit Wasser und dem Wärme entwickelnden Salz und flüssigem Polyalkohol zwischen 1 : 2 und 1 : 6 liegt.

11. Mittel nach einem der vorhergehenden Ansprüche, dadurch gekennzeichnet, daß es zusätzlich mindestens einen haarpflegenden Wirkstoff enthält.

12. Mittel nach Anspruch 11, dadurch gekennzeichnet, daß es einen Filmbildner enthält.

13. Mittel nach Anspruch 11, dadurch gekennzeichnet, daß es eine haarkonditionierende Substanz enthält.

14. Mittel nach Anspruch 11, dadurch gekennzeichnet, daß es natürliche und synthetische Fette und Öle enthält.

15. Mittel nach einem der vorhergehenden Ansprüche dadurch gekennzeichnet, daß die den Polyalkohol enthaltende Zusammensetzung (B) einen Penetrationspromoter enthält.

16. Mittel nach Anspruch 15, dadurch gekennzeichnet, daß es als Penetrationspromotor 1,3-Butandiol, 1,4-Butandiol, Benzylalkohol und/oder Benzyloxyethanol enthält.

17. Verfahren zur Haarbehandlung, dadurch gekennzeichnet, daß eine wasserfreie, ein physiologisch verträgliches, beim Zusammenbringen mit Wasser Wärme entwickelndes Salz und mindestens ein Verdickungsmittel enthaltende Zusammensetzung (A) nach den Ansprüchen 1 bis 14 mit Wasser und einer mindestens einen bei 25 °C flüssigen Polyalkohol aus der Gruppe Polyethylenglycol, Polypropylenglycol und Di-, Tri- oder Polyglycerin enthaltenden Zusammensetzung (B) nach den Ansprüchen 1 bis 16 unter Wärmeentwicklung vermischt, das entstandene Gel auf menschliches Haar aufgebracht und nach der Einwirkung auf das Haar ausgespült wird.

## Revendications

1. Composition pour le traitement de cheveux humains, comprenant au moins deux compositions conservées séparément jusqu'à l'application qui conduisent à une augmentation de la viscosité après mélange en présence d'eau en dégageant de la chaleur, dans laquelle une composition (A) contient un mélange essentiellement anhydre d'un sel physiologiquement compatible dégageant de la chaleur lorsque mélangé à l'eau, et au moins un agent épaississant, et une autre composition (B) maintenue séparée jusqu'à l'application contient au moins un polyalcool qui est liquide à 25°C choisi dans le groupe des polyéthylène glycols, polypropylène glycols, des glycérols et (ou) des diglycérols.

2. Composition selon la revendication 1, caractérisé en ce qu'elle contient un chlorure de métal bivalent physiologiquement compatible qui dégage de la chaleur lorsqu'il est mélangé à l'eau.

3. Composition selon la revendication 2, caractérisée en ce qu'elle contient du chlorure de calcium, du chlorure de magnésium et (ou) du chlorure de zinc comme chlorure de métal bivalent.

4. Composition selon l'une des revendications précédentes, caractérisée en ce que la composition (B) contient un polyéthylèneglycol ayant un poids moléculaire entre 200 et 600.

5. Composition selon la revendication 4, caractérisée en ce que la composition (B) contient un polyéthylèneglycol ayant un poids moléculaire de 400 à 600.

6. Composition selon l'une des revendications précédentes, caractérisée en ce qu'elle contient comme agent épaississant un dérivé de cellulose et (ou) un dérivé de gomme naturelle.

7. Composition selon l'une des revendications 1 à 5, caractérisée en ce qu'elle contient comme agent épaississant de la silice et (ou) un silicate d'aluminium.

8. Composition selon la revendication 6, caractérisée en ce qu'elle contient comme agent épaississant de la gomme xanthane et (ou) de la gomme guar.

9. Composition selon l'une des revendications précédentes, caractérisée en ce que, dans le mélange final à appliquer sur les cheveux, le rapport du sel physiologiquement compatible dégageant de la chaleur lorsque mélangé à l'eau au polyalcool liquide est entre 1/1 et 1/8.

10. Composition selon la revendication 9, caractérisée en ce le rapport en poids de sel dégageant de la chaleur lorsque mélangé à l'eau au polyalcool liquide est entre 1/2 et 1/6.

11. Composition selon l'une des revendications précédentes, caractérisée en ce qu'elle contient en outre au moins un agent de traitement des cheveux.

12. Composition selon la revendication 11, caractérisée en ce qu'elle contient un agent filmogène.

13. Composition selon la revendication 11, caractérisée en ce qu'elle contient une substance pour améliorer les cheveux.

14. Composition selon la revendication 11, caractérisée en ce qu'elle contient des graisses et des huiles synthétiques et naturelles.

15. Composition selon l'une des revendications précédentes, caractérisée en ce que la composition (B) qui contient un polyalcool comprend un promoteur de pénétration.

16. Composition selon la revendication 15, caractérisé' en ce qu'elle contient du 1,3-butanediol, du 1,4-butanediol, de l'alcool benzylique et (ou) du benzyloxyéthanol comme promoteur de pénétration.

17. Procédé pour le traitement de cheveux, caractérisé en ce qu'une composition (A) essentiellement anhydre contenant un sel physiologiquement compatible qui dégage de la chaleur lorsque mélangé à l'eau et au moins un agent épaississant, est mélangée avec une composition (B) contenant au moins un poly alcool qui est liquide à 25°C et qui est choisi dans le groupe des polyéthylèneglycols, des polypropylèneglycols et des di-, tri- ou poylglycérols selon les revendications 1 à 16, dégageant de la chaleur avec une augmentation de la viscosité, après quoi le gel ainsi formé est appliqué sur des cheveux humains et rincé après traitement.
